# FASCICULE DE BREVET EUROPEEN

(11) **EP 1 513 446 B1**
(45) Date de publication et mention de la délivrance du brevet: **29.03.2006**
(21) Numéro de dépôt: 03752837.9
(22) Date de dépôt: 16.05.2003
(51) Int. Cl.: A61B 5/103, G02B 21/00

(54) **DISPOSITIF DE TOMOBIOPSIE OPTIQUE CONFOCALE IN VIVO**
VORRICHTUNG FÜR EINEN IN VIVO KONFOKALEN OPTISCHEN TOMOGRAPHIESCHNITT
DEVICE FOR IN VIVO CONFOCAL OPTICAL TOMOGRAPHIC SECTION

(30) Priorité: 17.05.2002 FR 0206132
(43) Date de publication de la demande: 16.03.2005
(73) Titulaire: Biophymed, 34083 Montpellier Cedex 4 (FR)
(72) Inventeur: AGOPIAN, Lise, F-34170 Castelnau Le Lez (FR); PARIS, Jean-Pierre, F-34980 Saint Gelly Du Fesc (FR); DENIS, Bernard, F-30250 Aubais (FR)
(74) Mandataire: Gosse, Michel
(86) Numéro de dépôt international: PCT/FR2003/001514
(87) Numéro de publication internationale: WO 2003/096900

(56) Documents cités:
- FR-A- 2 738 140
- FR-A- 2 738 343

## Description

L'invention concerne un dispositif de tomobiopsie optique confocale in vivo destiné à l'acquisition, la reconstruction, la visualisation et l'enregistrement d'images instantanées d'un volume bien déterminé d'observation, notamment de peau et de ses microstructures.

Dans la pratique clinique, il est souvent nécessaire d'observer, en un instantané et de façon non invasive, les microstructures présentes dans les premières centaines de microns, voire les premiers millimètres, de profondeur et plus généralement d'examiner, en une série d'instantanés correspondant à une succession de plans de coupe, un élément de volume donné afin d'en caractériser la structure interne et/ou la composition.
En effet, l'imagerie cutanée in vivo revêt un attrait majeur puisqu'elle permet d'affiner certains diagnostics, de préciser les limites de certains processus pathologiques et enfin de contribuer à la progression des connaissances en physiologie de la peau.
La tomobiopsie optique cutanée in vivo constitue un progrès par rapport à l'existant dans les domaines de la recherche fondamentale, de la recherche pharmaceutique et de la recherche cosmétologique.

L'analyse de l'état de l'art en matière d'observation des microstructures cutanées met en évidence l'existence de cinq procédés basés sur des principes physiques différents, à savoir :
- la microscopie optique "conventionnelle" permettant l'observation d'échantillons issus de biopsies cliniques ;
- la microscopie électronique permettant l'observation d'échantillons issus de biopsies cliniques et spécialement traités pour ce type d'examen ;
- l'échographie ultrasonore permettant l'observation non invasive de plans de coupe parallèles à la direction de propagation de l'onde d'excitation ;
- la tomobiopsie optique, à fente de filtrage, permettant l'observation non invasive de plans de coupe parallèles entre eux et parallèles à l'axe optique du système (brevet FR-2738140) ;
- la microstratigraphie optique, confocale, permettant l'observation non invasive au moyen d'un objectif, à chromatisme axial connu, formant un ensemble d'images monochromatiques définissant un segment de droite (brevet FR-2738343).
Parmi ces cinq procédés :
- les microscopies optique et électronique sont invasives et destructives ;
- l'échographie ultrasonore n'offre pas une résolution et une précision de mesure qui soient compatibles avec les besoins de nombreuses applications ;
- la tomobiopsie optique par fente de filtrage (ligne lumineuse) et par utilisation de lumière blanche, présente les limitations suivantes : phénomène de "diaphonie" entre les divers canaux de l'image avec limitation de la profondeur d'observation (0,3 à 0,4 mm maximum) ; en outre, le procédé utilisé pour la mise en oeuvre d'une biopsie optique est mal adapté à la prise d'image en milieu biologique ;
- la microstratigraphie optique confocale ne permet pas l'observation simultanée et instantanée de structures internes situées à des profondeurs distinctes ; en outre, de par les sources de lumière utilisées (lumière blanche et faibles puissances), elle présente la limitation liée aux profondeurs d'observation (0,5 mm maximum).

Le dispositif selon l'invention a été conçu pour :
- d'une part, éliminer les inconvénients susmentionnés et plus particulièrement atteindre, voire dépasser, des profondeurs d'observation de 3 mm englobant donc l'épiderme et le derme profond ;
- d'autre part, supprimer des sous-ensembles importants de la voie d'observation, à savoir : le scanner et l'objectif habituellement utilisés dans les techniques optiques susmentionnées.

Le dispositif selon l'invention comporte généralement :
a) une voie d'éclairage adaptée pour illuminer la zone sous examen selon une technique de coupe tomographique bien spécifique et constituée, successivement, d'une source de lumière, d'une première fibre optique, d'un coupleur à fibres optiques, à deux voies, pourvu d'un premier port de sortie relié à une deuxième fibre optique dont l'extrémité libre joue le rôle de source ponctuelle pour un dispositif adapté pour, d'une part, former, dans la zone de tomobiopsie optique, un segment de droite d'observation constitué d'un ensemble d'images définies par leur longueur d'onde et leur distance à la surface d'observation, et, d'autre part, déplacer, dans ladite zone d'observation, selon une trajectoire bien définie, ledit segment de droite ; b) une voie d'observation permettant de recueillir, selon le principe du retour inverse de la lumière, le flux rétrodiffusé par les détails internes de la zone de tomobiopsie optique illuminée par la voie d'éclairage, et constituée, successivement, du dispositif destiné à former et à déplacer le segment de droite d'observation, de la deuxième fibre optique, du coupleur à fibres optiques qui est pourvu d'un deuxième port de sortie relié à une troisième fibre optique reliée elle même à un sous ensemble de dispersion chromatique, du type spectrophotomètre, qui analyse la lumière diffusée par les segments d'observations successifs, d'une unité d'acquisition et de visualisation, du type détecteur photoélectrique linéaire ou matriciel bidimensionnel, placée dans le plan image dudit spectrophotomètre et délivrant, à l'aide de son électronique de pilotage, une image instantanée de la zone de tomobiopsie selon une fréquence de rafraîchissement bien définie, d'une liaison de synchronisation reliant ladite unité d'acquisition et de visualisation au dispositif destiné à former et à déplacer le segment de droite d'observation.
Le dispositif selon l'invention se caractérise essentiellement en ce que :
- le dispositif adapté pour former, dans la zone de tomobiopsie optique, un segment de droite d'observation constitué d'un ensemble d'images définies par leur longueur d'onde et leur distance à la surface d'observation, est réalisé au moyen de l'extrémité de la deuxième fibre optique qui est positionnée à proximité de la zone d'observation ;
- le dispositif adapté pour déplacer, dans ladite zone d'observation, selon une trajectoire bien définie, ledit segment de droite, est réalisé au moyen d'un organe électromécanique adapté pour déplacer ladite extrémité le long de la surface de la zone d'observation et selon ladite trajectoire.
Le scanner et l'objectif ont donc été remplacés par l'organe électromécanique destiné à déplacer, sur la zone d'observation, l'extrémité de la deuxième fibre optique.
Il se caractérise également en ce que
- l'organe électromécanique est adapté pour déplacer l'extrémité de la deuxième fibre optique, selon une trajectoire hélicoïdale balayant un volume d'observation de diamètre et de profondeur donnés ;
- l'unité d'acquisition et de visualisation est programmée pour obtenir une représentation graphique déroulée des images successives des segments de droite d'observation du volume d'observation extrait.

Les caractéristiques et les avantages de l'invention vont apparaître plus clairement à la lecture de la description détaillée qui suit d'au moins un mode de réalisation préféré de celle-ci donné à titre d'exemple non limitatif et représenté aux dessins annexés.
Sur ces dessins :

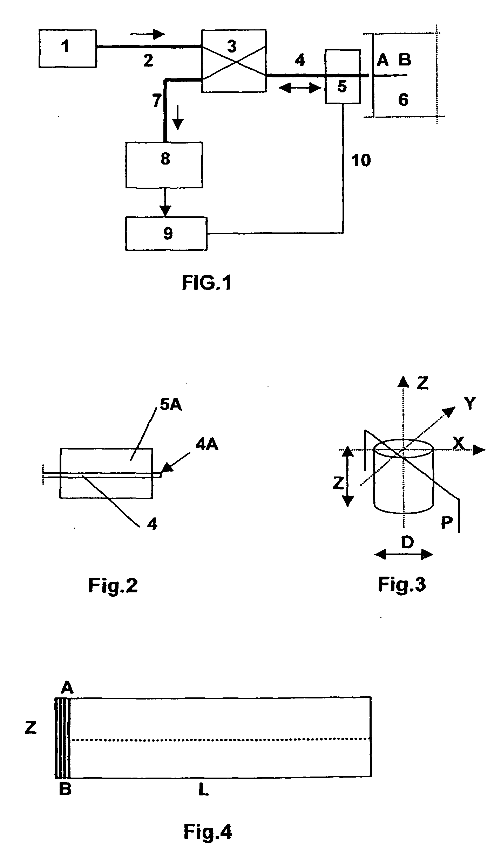

- la figure 1 est un schéma de principe du dispositif selon l'invention ;
- la figure 2 est un schéma de détail de l'extrémité de la deuxième fibre et de l'organe électromécanique associé ;.
- la figure 3 est un schéma représentant un volume de tomobiopsie optique obtenu par balayage hélicoïdal ;
- la figure 4 est un schéma représentant ledit volume déroulé sur un plan.

Le dispositif représenté aux figures comporte :
a) une voie d'éclairage adaptée pour illuminer la zone sous examen selon une technique de coupe tomographique bien spécifique et constituée, successivement, d'une source de lumière (1), d'une première fibre optique (2), d'un coupleur à fibres optiques (3), à deux voies, pourvu d'un premier port de sortie relié à une deuxième fibre optique (4) dont l'extrémité (4A) joue le rôle de source ponctuelle pour un dispositif (5) adapté pour, d'une part, former, dans la zone de tomobiopsie optique (6), un segment de droite d'observation (AB) constitué d'un ensemble d'images définies par leur longueur d'onde et leur distance (Z) à la surface d'observation, et, d'autre part, déplacer, dans ladite zone d'observation (6), selon une trajectoire (X,Y) bien définie, ledit segment de droite (AB) ;
b) une voie d'observation permettant de recueillir, selon le principe du retour inverse de la lumière, le flux rétrodiffusé par les détails internes de la zone de tomobiopsie optique (6) illuminée par la voie d'éclairage, et constituée, successivement, du dispositif (5) destiné à former et à déplacer le segment de droite (AB), de la deuxième fibre optique (4), du coupleur à fibres optiques (3) pourvu d'un deuxième port de sortie relié à une troisième fibre optique (7) reliée elle même à un sous ensemble de dispersion chromatique (8), du type spectrophotomètre, qui analyse la lumière diffusée par les segments d'observations successifs (AB), d'une unité d'acquisition et de visualisation (9), du type détecteur photoélectrique linéaire ou matriciel bidimensionnel, placée dans le plan image dudit spectrophotomètre et délivrant, à l'aide de son électronique de pilotage, une image instantanée de la zone de tomobiopsie selon une fréquence de rafraîchissement bien définie, d'une liaison de synchronisation (10) reliant ladite unité d'acquisition et de visualisation (9) au dispositif (5) destiné à former et à déplacer le segment de droite d'observation (AB) ;

Il comporte plus particulièrement :
a) comme source de lumière: un ensemble constitué de la source de lumière ponctuelle (1) et de la première fibre optique (2) dont la longueur est définie pour obtenir globalement un spectre lumineux s'étendant de la lumière blanche à l'infrarouge ;
b) comme dispositif (5) destiné à former et à déplacer le segment de droite d'observation (AB) : un ensemble constitué de l'extrémité (4A) de la deuxième fibre optique (4) qui est positionnée à proximité de la zone d'observation (6) et d'un organe électromécanique (5A) adapté pour déplacer ladite extrémité selon une trajectoire (X,Y) correspondant au balayage souhaité de ladite zone.
Selon d'autres particularités de réalisation dudit dispositif :
- la source de lumière (1) est un générateur de lumière cohérente dont la longueur d'onde est égale à 532 nm ;
- la première fibre optique (2) est une fibre dont la longueur est définie pour obtenir un spectre couvrant au moins la plage de longueurs d'ondes comprises entre 500 et 1300 nm ;
- l'organe électromécanique (5A) entraînant l'extrémité (4A) de la deuxième fibre optique (4), est adapté pour donner à cette dernière une trajectoire hélicoïdale de manière à balayer une volume d'observation de diamètre (D) et de profondeur (Z) bien définis :

- l'unité d'acquisition et de visualisation (9) est programmée pour obtenir une représentation graphique déroulée (Z,L) des images successives des segments de droite d'observation (AB) du volume d'observation (D,Z) extrait;
- l'unité d'acquisition et de visualisation (9) est programmée pour obtenir une représentation graphique des segments de droite d'observation (AB) constitutifs de plans de coupe (P) effectués à l'intérieur du volume d'observation (D,Z) extrait.
Selon d'autres particularités de réalisation dudit dispositif :
- la source(1), qui émet une lumière cohérente dont la longueur d'onde est égale à 532 nm, est réalisée au moyen d'un laser ou d'une diode laser doublée en fréquence par un cristal non linéaire placé en cavité interne de longueur d'onde 1064 nm ;
- les fibres optiques (2), (4) et (7), le coupleur (3) et tous les moyens de liaison (connectique) desdits éléments entre eux, sont de type standard ;
- la fibre optique (2) peut être éventuellement du type photonique avec coupleurs optiques d'entrée et de sortie adaptés.
A titre d'exemple :
- dans le cas de l'utilisation d'une fibre optique standard (∅ 50/125 µm): une longueur de 500 à 600 m est nécessaire pour obtenir un continuum de lumière blanche du vert jusqu'à l'infrarouge profond de longueur d'onde au moins égale à 1300 nm, et d'atteindre une profondeur d'observation de 2 à 3 mm, la longueur de cette fibre détermine la limite de la longueur d'onde dans l'infrarouge ;
- dans le cas de l'utilisation d'une fibre optique photonique (∅ coeur = 1,6 µm) : une longueur de 1,50 m est nécessaire pour obtenir un continuum de lumière blanche du vert jusqu'au proche infrarouge de longueur d'onde de 800 nm, et d'atteindre une profondeur d'observation de 500 à 800 µm.
Le micro-laser est pulsé avec des fréquences compatibles avec la fibre optique. Les impulsions peuvent être très courtes de quelques 10 fentos à quelques picos secondes et les fréquences des impulsions peuvent se situer entre 5 et 100 MHz.
Le coupleur optique (2) est d'un type standard achromatique.
La caméra de l'unité d'acquisition et de visualisation est à très haute résolution et dynamique (100 dB).
La source de lumière (1) présente l'avantage de pouvoir émettre un continuum de lumière dans une fibre optique avec une intensité dépassant de plusieurs ordres de grandeurs les niveaux d'intensité des autres sources de lumière connues.
Le coupleur (3) est achromatique et est du type 50:50.
Le spectrophotomètre ou analyseur de spectre optique (8) permet l'analyse de la lumière diffusée par l'objet. Il utilise un élément dispersif comme un prisme ou un réseau de diffraction.
On peut par exemple effectuer une prise de mesure en mode A-scan avec une prise d'image à cadence vidéo soit de 25 images par seconde pour une caméra matricielle. On dispose alors de 40 ms pour construire une image. Par exemple, en supposant que le temps d'intégration d'un seul spectre en mode A-scan soit de 1 ms, il est possible alors de réaliser 40 tirs.
Si la taille du spot d'analyse est de 3 µm on disposera d'une image au format 120xZ µm² (où Z est la profondeur de l'analyse).
Pour un temps d'intégration de 400ms, le format de l'image sera de 1200xZ µm².
On constate ainsi que le critère de temps d'acquisition en mode A-scan est fondamental pour atteindre l'objectif de fonctionnement in-vivo.
Il est utile de mentionner l'existence de plusieurs facteurs qui limitent une prise de mesure rapide :
- l'Eclairement Maximal Permis (EMP) qui fixe le seuil d'éclairement maximal que le tissu cutané peut supporter ;
- la puissance des sources à spectre large disponibles sur le marché ;
- le bruit électronique des détecteurs et de l'électronique de lecture qui fixe le nombre minimal de photons devant tomber sur le détecteur permettant de générer un nombre de photon-électrons suffisant pour que le signal sorte du bruit ;
- la dynamique des signaux optiques qui est très importante et qui impose de travailler avec des détecteurs de 100 dB minimum pour des profondeurs d'analyse de l'ordre de quelques mm.
La résolution dans le plan de l'objet, ou résolution latérale, dépendra de la fibre optique choisie ainsi que de la combinaison optique retenue.
Les coupleurs achromatiques disponibles sur le marché sont élaborés pour des fibres multimodes du type 50/125 µm ouvertes à 0,2. Il est bien sûr possible d'utiliser d'autres types de coupleurs.

## Revendications

1. Dispositif optoélectronique de tomobiopsie optique confocale in vivo, destiné à l'acquisition, la reconstruction, la visualisation et l'enregistrement d'images instantanées d'un volume bien déterminé d'observation, notamment de peau et de ses microstructures, comportant généralement :
a) une voie d'éclairage adaptée pour illuminer la zone sous examen selon une technique de coupe tomographique bien spécifique et constituée, successivement, d'une source de lumière (1), d'une première fibre optique (2), d'un coupleur à fibres optiques (3), à deux voies, pourvu d'un premier port de sortie relié à une deuxième fibre optique (4) dont l'extrémité (4A) joue le rôle de source ponctuelle pour un dispositif (5) adapté pour, d'une part, former, dans la zone de tomobiopsie optique (6), un segment de droite d'observation (AB) constitué d'un ensemble d'images définies par leur longueur d'onde et leur distance (Z) à la surface d'observation, et, d'autre part, déplacer, dans ladite zone d'observation (6), selon une trajectoire (X,Y) bien définie, ledit segment de droite (AB) ;
b) une voie d'observation permettant de recueillir, selon le principe du retour inverse de la lumière, le flux rétrodiffusé par les détails internes de la zone de tomobiopsie optique (6) illuminée par la voie d'éclairage, et constituée, successivement, du dispositif (5) destiné à former et à déplacer le segment de droite (AB), de la deuxième fibre optique (4), du coupleur à fibres optiques (3) pourvu d'un deuxième port de sortie relié à une troisième fibre optique (7) reliée elle même à un sous ensemble de dispersion chromatique (8), du type spectrophotomètre, qui analyse la lumière diffusée par les segments d'observations successifs (AB), d'une unité d'acquisition et de visualisation (9), du type détecteur photoélectrique linéaire ou matriciel bidimensionnel, placée dans le plan image dudit spectrophotomètre et délivrant, à l'aide de son électronique de pilotage, une image instantanée de la zone de tomobiopsie selon une fréquence de rafraîchissement bien définie, d'une liaison de synchronisation (10) reliant ladite unité d'acquisition et de visualisation (9) au dispositif (5) destiné à former et à déplacer le segment de droite d'observation (AB) ; **caractérisé en ce que** le dispositif adapté pour former, dans la zone de tomobiopsie optique (6), un segment de droite d'observation (AB) constitué d'un ensemble d'images définies par leur longueur d'onde et leur distance (Z) à la surface d'observation, est réalisé au moyen de l'extrémité (4A) de la deuxième fibre optique (4) qui est positionnée à proximité de la zone d'observation (6) et **en ce que** le dispositif adapté pour déplacer, dans ladite zone d'observation (6), selon une trajectoire (X,Y) bien définie, ledit segment de droite (AB), est réalisé au moyen d'un organe électromécanique (5A) adapté pour déplacer ladite extrémité (4A) le long de la surface de la zone d'observation (6) et selon ladite trajectoire (X,Y).

2. Dispositif, selon la revendication 1, **caractérisé en ce que** l'organe électromécanique (5A) est adapté pour déplacer l'extrémité (4A) de la deuxième fibre optique (4), selon une trajectoire hélicoïdale balayant un volume d'observation de diamètre (D) et de profondeur (Z).

3. Dispositif, selon la revendication 1, **caractérisé en ce que** l'unité d'acquisition et de visualisation (9), est programmée pour obtenir une représentation graphique déroulée (Z,θ) des images successives des segments de droite d'observation (AB) du volume d'observation (D,Z) extrait.

4. Dispositif, selon la revendication 1, **caractérisé en ce que** l'unité d'acquisition et de visualisation (9) est programmée pour obtenir une représentation graphique des segments de droite d'observation (AB) constitutifs de plans de coupe (P) effectués à l'intérieur du volume d'observation (D,Z) extrait.

5. Dispositif, selon la revendication 1, **caractérisé en ce que** la source de lumière (1) est réalisée au moyen d'un laser ou d'une diode laser de longueur d'onde 532 nm, doublée en fréquence par un cristal non linéaire placé en cavité interne de longueur d'onde 1064 nm et de la première fibre optique (2), de type standard, dont la longueur est définie pour obtenir un continuum de lumière blanche jusqu'à une longueur d'onde au moins égale à 1 300 nm.

6. Dispositif, selon la revendication 1, **caractérisé en ce que** la source de lumière (1) est réalisée au moyen d'un laser ou d'une diode laser de longueur d'onde 532 nm, doublée en fréquence par un cristal non linéaire placé en cavité interne de longueur d'onde 1064 nm et de la première fibre optique (2), de type photonique, dont la longueur est définie pour obtenir un continuum de lumière jusqu'à une longueur d'onde égale à 800 nm.

## Claims

1. Optoelectronic device for confocal optical tomobiopsy in vivo designed to capture, reconstruct, display and record instant images from a well determined observation volume, namely of skin and its microstructures, generally composed of:
a) a lighting path adapted for lighting the area being examined according to a technique of well-specific tomography cut and successively composed of a light source (1), a first optical fibre (2), a two-way optical fibre coupler (3) provided with a first output port connected to a second optical fibre (4) the end (4A) of which acts as punctual source for a device (5) adapted for, on the one hand, forming within the optical tomobiopsy area (6) a right observation segment (AB) constituted of a set of images defined by their wavelength and their distance (Z) on the observation surface and, on the other hand, moving, in the said observation area (6) along a well-defined path (X, Y), the said right segment (AB);
b) an observation path allowing to collect, according to be principle of the inverted light return, the flux back diffused by the internal details of the optical tomobiopsy area (6) lighted by the lighting path and successively constituted of the device (5) designed to form and move the right segment (AB), the second optical fibre (4), the optical fibre coupler (3) provided with a second output port connected to a third optical fibre (7) in turn connected to a subassembly of chromatic dispersion (8) of the spectrophotometer type, which analyses the light diffused by the successive observation segments (AB) of a unit for capturing and displaying (9), of the linear photoelectric type or twedimensional matrix type, placed in the image plane of the said spectrophotometer and which releases, with the assistance of electrosonic control, an instant image of the tomobiopsy area according to a well defined refresh rate, from a synchronisation connection (10) connecting the said capture and display unit (9) to the device (5) designed to form and to move the right observation segment (AB), **characterised in that** the device adapted to form in the optical tomobiopsy area (6) a right observation segment (AB) constituted of a set of images defined by their wavelength and their distance (Z) to the observation surface is made by means of the end (4A) of the second optic fibre (4) which is placed near to the observation area (6) and **in that** the device adapted for moving, in the said observation area (6) according to a well defined path (X,Y), the said right segment (AB), is made by means of an electromechanical organ (5A) adapted for moving the said end (4A) along the surface of the observation area (6) and along the said path (X, Y).

2. Device, according to claim 1, **characterised in that** the electromechanical organ (5A) is adapted for moving the end (4A) of the second optical fibre (4) according to a helical path scanning an observation volume having a (D) diameter and (Z) depth.

3. Device, according to claim 1, **characterised in that** the capture and display unit (9) is programmed to obtain a spread schematic diagram (Z,0), of the successive images of the right observation segments (AB) of the observation volume (DZ) achieved.

4. Device according to claim 1, **characterised in that** the unit to capture and display (9) is programmed to obtain a display of the right observation segments (AB) constituting cutting planes (P) carried out within the observation volume (D,Z) achieved.

5. Device according to claim 1, **characterised in that** the light source (1) is made of a laser or a laser diode having a wavelength of 532 nm, doubled in frequency by a non linear crystal placed in the internal recess having a wavelength of 1064 nm and of the standard type first optical fibre (2), the length of which is defined to obtain a continuumof white light up to a wavelength at least equal to 1 300 nm.

6. Device according to claim 1, **characterised in that** the light source (1) is made of a laser or laser diode having a wavelength of 532 nm, doubled in frequency by a non linear crystal placed in the internal cavity having a wavelength of 1064 nm and of the first optical fibre (2) of the photon type, the length of which is defined to obtain a continuum of light up to a wavelength equal to 800 nm.

## Patentansprüche

1. Optoelektronische Vorrichtung zur konfokalen, optischen Moment-Tomo-Biopsie für die Aufnahme, die Wiederherstellung, die Anzeige und die Aufzeichnung von Momentaufnahmen eines klar definierten Beobachtungsvolumens, besonders der Haut und deren Mikrostrukturen, die allgemein aus folgenden Bauteilen besteht:
a) einer angepassten Beleuchtungslinie für die Beleuchtung des zu untersuchenden Bereichs mittels einer eindeutig spezifizierten und ausgebildeten tomographischen Schnitttechnik, weitehin einer Lichtquelle (1), einer ersten optischen Faser (2) einem 2-poligen Anschluss für optische Fasern (3) mit einem ersten Ausgangs-Port, der mit einer zweiten optischen Faser (4) verbunden ist, dessen Ende (4A) die Aufgabe der PunktQuelle für eine Vorrichtung (5) erfüllt, die darauf ausgelegt ist, um einerseits im Bereich der optischen Tomo-Biopsie (6) ein rechtes Beobachtungssegment (AB) zu bilden, das aus einer Einheit von Bildern gebildet wird, die durch ihre Wellenlänge und ihrem Abstand (Z) zur Beobachtungsfläche definiert werden, und um andererseits das rechte Segment (AB) im benannten Beobachtungsbereich (6) gemäß einem eindeutig definierten Streckenverlauf (X, Y) zu bewegen;
b) einer Beobachtungslinie, mit der mittels des Prinzips der Lichtreflektierung der Lichtfluss aufgenommen wird, der von den internen Details des Bereichs der optischen Tomo-Biopsie (6) reflektiert wird, der durch die Beleuchtungslinie beleuchtet wird; nachfolgend besteht sie aus der Vorrichtung (5), die das rechte Segment (AB) bildet und verschiebt, der zweiten optischen Faser (4), dem Anschluss für optische Fasern (3) mit einem zweiten Ausgangs-Port, der mit einer dritten optischen Faser (7) verbunden ist, die wiederum mit einer Untereinheit für die chromatische Verteilung(8) verbunden ist, Typ Spektralfotometer, der das von den aufeinander folgenden Beobachtungssegmenten (AB) verteilte Licht untersucht, einer Aufzeichnungs und Anzeigeeinheit (9), Typ linearer fotoelektrischer Detektor oder zweidimensionaler Matrixdetekta, der auf der Bildebene des Spektralfotometers angeordnet ist, und der mittels seiner Steuerelektronik und mit einer definierten Auffrischungsrate eine Momentaufnahme des Tomo Biopsiebereichs liefert, einer Synchronisationsverbindung (10) zur Verbindung der angeführten Aufzeichnungs- und Anzeigeeinheit (9) mit der Vorrichtung (5) zur Bildung und Verschiebung des Beobachtungssegments (AB), **dadurch gekennzeichnet, dass** die Vorrichtung zur Bildung eines Beobachtungssegments (AB) im optischen Tomo-Biopsiebereich (6) gebildet aus einer Reihe von Bildern, die durch ihre Wellenlänge und ihrem Abstand (Z) zur Beobachtungsfläche definiert werden, mittels des Endes (4A) der zweiten optischen Faser (4) gebildet wird, die in der Nähe des Beobachtungsbereichs (6) angebracht wird, und dass die Vorrichtung zur Verschiebung des angeführten rechten Segments (AB) im Beobachtungsbereich (6) entsprechend einer definierten Strecke (X, Y) mittels eines elektromechanischen Elements (5A) durchgeführt wird, das zur Verschiebung des Endes (4A) entlang der Oberfläche des Beobachtungsbereichs (6) und entlang der angeführten Strecke (X, Y) angepasst ist.

2. Vorrichtung gemäß dem Patentanspruch 1, **dadurch gekennzeichnet, dass** das elektromechanische Element (5A) dafür angepasst ist, das Ende (4A) der zweiten optischen Faser (4) über eine spiralförmige Strecke zu bewegen und dabei ein Beobachtungsvolumen mit Durchmesser (D) und einer Tiefe (Z) beleuchtet.

3. Vorrichtung gemäß dem Patentanspruch 1, **dadurch gekennzeichnet, dass** die Aufzeichnungs- und Anzeigeeinheit (9) dafür programmiert ist, eine graphische Darstellung (Z,0) aufeinander folgender Bilder der rechten Beobachtungssegmente (AB) des Beobachtungsvolumens (D,Z) zu erhalten.

4. Vorrichtung gemäß dem Patentanspruch 1, **dadurch gekennzeichnet, dass** die Aufzeichnungs- und Anzeigeeinheit (9) dafür programmiert ist, eine graphische Darstellung der rechten Beobachtungssegmente (AB) zu erhalten, die aus den Schnittebenen (P) gebildet werden, die im Innem des Beobachtungsvolumens (D,Z) angefertigt werden.

5. Vorrichtung gemäß dem Patentanspruch 1, **dadurch gekennzeichnet, dass** die Lichtquelle (1) aus einem Laser oder einer Laserdiode mit einer Wellenlänge von 532 nm gebildet wird, dessen Frequenz durch ein in den inneren Hohlraum eingesetztes nicht lineares Kristall auf eine Wellenlänge 1064 nm verdoppelt wird, und aus der ersten optischen Faser (2), in Standardausführung, wobei die Wellenlänge definiert ist, um ein kontinuierliches weißes Licht bis zu einer Wellenlänge von mindestens 1300 nm zu erhalten.

6. Vorrichtung gemäß dem Patentanspruch 1, **dadurch gekennzeichnet, dass** die Lichtquelle (1) aus einem Laser oder einer Laserdiode mit einer Wellenlänge von 532 nm, dessen Frequenz durch ein eingesetztes nicht lineares Kristall auf eine Wellenlänge 1064 nm verdoppelt wird, und aus der ersten optischen Faser (2) gebildet wird, in photonischer Ausführung, wobei die Wellenlänge definiert ist, um ein kontinuierliches weißes Licht bis zu einer Wellenlänge von 800 nm zu erhalten.
